# EUROPEAN PATENT APPLICATION

(11) **EP 3 210 605 A1**
(43) Date of publication of application: **30.08.2017**
(21) Application number: 17165100.3
(22) Date of filing: 08.11.2006
(51) Int. Cl.: A61K 31/4174, A61K 31/4439, A61K 31/444, A61K 31/50, A61K 31/501, A61P 9/04

(54) **USE OF PDE III INHIBITORS FOR THE TREAMENT OF ASYMPTOMATIC (OCCULT) HEART FAILURE**

(30) Priority: 14.11.2005 EP 05110689
(62) Divisional of application: 06829958.5
(71) Applicant: Boehringer Ingelheim Vetmedica GmbH, 55216 Ingelheim am Rhein (DE)
(72) Inventor: KLEEMANN, Rainer, 55216 Ingelheim am Rhein (DE); TROETSCHEL, Christian, 55216 Ingelheim am Rhein (DE); VOLLMAR, Andrea, 57537 Wissen (DE)
(74) Representative: Simon, Elke Anna Maria

(57) **Abstract**

The invention relates to the use of a phosphodiesterase type III (PDE III) inhibitor and/or Ca²⁺-sensitizing agent or a pharmaceutically acceptable derivative thereof for the preparation of a medication for the prolongation of time until onset of clinical symptoms in patients having an asymptomatic (occult) heart failure and/or for the reduction of heart size in patients having an asymptomatic (occult) heart failure.

## Description

### BACKGROUND OF THE INVENTION

### 1. TECHNICAL FIELD

The invention relates to the use of a phosphodiesterase type III (PDE III) inhibitor or a "Ca²⁺-sensitizing agent" or a pharmaceutically acceptable derivative thereof for the preparation of a medication for the reverse remodelling of the heart of a patient with asymptomatic (occult) heart failure and their delayed onset of clinical symptoms.

### 2. BACKGROUND INFORMATION

Heart failure is divided in different stages (ISACHC - Stages - Class I - III). The pathology of the heart begins with ISACHC Class I in which cardiac murmur or cardiac chamber enlargement, but no clinical symptoms are present (Class I or occult stage). It is known that the progress of heart failure is associated with an increase of the size of the heart. In dilated cardiomyopathy (DCM) the ratio of left ventricular wall thickness to chamber diameter is decreased and the circumferences of the annuluses of the mitral and tricuspid valves are increased in proportion to the magnitude of chamber dilation. DCM may either be caused primarily by e.g. genetic abnormalities ar secondarily e.g. due to valvular insufficiency both resulting in cardiac volume overload. However, it involves usually cardiac remodeling that may be defined as genome expression, molecular, cellular, and interstitial changes manifested clinically as changes in size, shape, and function of the heart. Cardiac remodelling is generally an adverse sign and linked to heart failure progression. Reverse cardiac remodelling is a goal of the treatment of heart failure therapy.

Heart failure therapy has traditionally focussed largely on symptomatic relief rather than on addressing underlying disease problems. Many dogs with symptomatic DCM have a very guarded prognosis (Monnet et al., 1995), with Dobermanns in particular generally experiencing only short survival times (Calvert et al., 1982; Calvert et al., 1997). There have been few studies examining the influence of treatment on survival in dogs with symptomatic DCM, although a subanalysis of the dogs with DCM in the LIVE study showed an improvement in time to treatment failure in those dogs receiving enalapril compared with placebo (142.8 versus 56.5 days, respectively) (Ettinger et al., 1998). On the whole, oral positive inotropic agents have lost favour in the treatment of chronic heart failure in human patients in recent years, after a number of trials revealed adverse effects on survival despite short-term hemodynamic benefits (Packer et al., 1991; Cowley and Skene, 1994). Recently it has been suggested that calcium sensitising agents may result in positive inotropic effects without producing some of the adverse effects (including calcium overload) associated with more traditional positive inotropes such as dobutamine, amrinone and milrinone.

In ISACHC Class I stage heart failure, in which cardiac murmur or cardiac chamber enlargement, but no clinical symptoms are present, therapy would have two objectives:
- reduce the pathologic changes of the dimensions of the heart (to the normal parameters - "reverse remodelling")
- prolong the time until onset of clinical symptoms

However, there is currently no drug available wich has proven to be efficacious in the reduction of the pathologic changes of the dimensions of the heart to the normal parameters "reverse remodelling", at asymptomatic stage (ISACHC Class I). A study has been published with the use of ACE - Inhibitors in the asymptomatic stage, but no therapeutic effect was shown (SVEP - Trial), neither a reverse remodelling nor a prolongation of the time until onset of clinical symptoms.

When the pathology progresses also clinical symptoms are present (Class II or III) and for this stage, several drugs showed a benefit in quality of life and some also in survival time. One of these drugs are phosphodiesterase type III (PDE III) inhibitors or "Ca²⁺-sensitizing agents", for example cilostazol, pimobendan, milrinone, levosimendan, amrinone, enoximone and piroximone TZC-5665, etc. Rather than increasing calcium entry into cardiac myocytes, calcium sensitisers achieve their positive inotropic effect by sensitising the contractile proteins to existing cytosolic calcium, by altering the binding of calcium with troponin-C. Producing a positive inotropic effect by calcium sensitising thereby avoids some of the adverse effects of cytosolic calcium overload. Increased cytosolic calcium levels have been associated with an increased tendency for arrhythmias and sudden death. Clinical trials of long-term use of oral pimobendan in human patients with heart failure have demonstrated an improvement in exercise tolerance and quality of life without significantly adverse effects on survival (Kubo et al., 1992; Katz et al., 1992).

The problem underlying the present invention was to provide a medication, which allows to increase the time without clinical symptoms and to remodel the size of the heart to reduce the risk of death in patients with asymptomatic heart disease (ISACHC Class I).

### BRIEF DESCRIPTION OF THE INVENTION

It has surprisingly been found that phosphodiesterase type III (PDE III) inhibitors and/or Ca²⁺-sensitizing agents, preferably pimobendan, or a pharmaceutically acceptable derivatives thereof can be used for the preparation of a medication able to prolong the time until onset of clinical symptoms and for the reduction of the heart size in cases of asymptomatic (occult) heart failure, e.g. asymptomatic DCM.

Moreover, the invention relates to a method of reduction of the heart size in cases of asymptomatic (occult) heart failure, e.g. asymptomatic DCM, which method comprises administering to a patient having an asymptomatic heart failure, e.g. asymptomatic DCM, an effective amount of an PDE III inhibitor, preferably pimobendan, or a pharmaceutically acceptable derivative thereof.

Furthermore, the invention relates to an article of manufacture comprising packaging material contained within which is a composition effective to prolong the time until onset of symptoms and to reduce the heart size of a patient having an asymptomatic (occult) heart failure, e.g. asymptomatic DCM, and the packaging material comprises a label which indicates that the composition can be used to prolong the time until onset of symptoms and to reduce the heart size of a patient having an asymptomatic (occult) heart failure, e.g. asymptomatic DCM, wherein said composition comprises at least one PDE III inhibitor or a Ca²⁺-sensitizing agent, preferably pimopendan, or a pharmaceutically acceptable derivative thereof.

### DETAILLED DESCRIPTION OF THE INVENTION

The invention relates to the use of a phosphodiesterase type III (PDE III) inhibitor, preferably a PDE III inhibitor, a Ca²⁺-sensitizing agent, or a PDE III inhibitor which exhibits additionally calcium sensitising effects (Ca²⁺-sensitizing agent) , or a pharmaceutically acceptable derivative thereof for the preparation of a medication for the prolongation of the time until onset of clinical symptoms of the heart disease and the reduction of the heart size of a patient having an asymptomatic (occult) heart failure, e.g. asymptomatic DCM.

The term "PDE III inhibitor" as used hereinabove and hereinbelow relates to phosphodiesterase (PDE) III inhibitors, which prevent breakdown of cAMP to 5'AMP and thus maintain the effect of cAMP on protein kinase and other secondary messenger activation.

The effects of PDE III inhibitors are as a rule positive inotropy and vasodilatation, which reduces the afterload patients with heart failure feel better.

The term Ca²⁺-sensitizing agent relates to a compound which increases the Ca²⁺ sensitivity of cardiac contractile proteins, i.e. increase the developed contractile force at a given concentration of Ca²⁺.

Preferred PDE III inhibitors or Ca²⁺-sensitizing agents are cilostazol, pimobendan, milrinone, levosimendan, amrinone, enoximone and piroximone TZC-5665 or pharmaceutically acceptable salts, derivatives, metabolites or pro-drugs thereof. Most preferred are pimobendan and levosimendan, or pharmaceutically acceptable salts, derivatives, metabolites or pro-drugs thereof . Even more preferred is pimobendan and levosimendan. Evenmore preffered is pimobendan, pharmaceutically acceptable salts, derivatives, metabolites or pro-drugs thereof.

Pimobendan, known to the public as 4,5-dihydro-6-[2-(4-methoxyphenyl)-1H-benzimidazol-5-yl]-5-methyl-3(2H)-pyridazone, is for example disclosed in EP 008 391 B1. Levosimendan is a pyridazone-dinitrile derivative. In particular, levosimendan is known to the public as (R)-[[4-(1,4,5,6-Tetrahydro-4-methyl-6-oxo-3-pyridazinyl)phenyl]hydra zono]propanedinitrile and has been described earlier for example in GB 2228004, US 5,151,420 and US 5,569,657.

The term "patient" as used hereinabove and hereinbelow relates to an animal or a personhaving an asymptomatic (occult) heart failure, e.g. asymptomatic DCM. The term "patient" embraces mammal such as primates including humans.

In addition to primates, a variety of other mammals can be treated according to the method of the present invention. For instance, mammals, including but not limited to, cows, sheep, goats, horses, dogs, cats, guinea pigs, rats or other bovine, ovine, equine, canine, feline, rodent or murine species can be treated. However, the method can also be practiced in other species, such as avian species.

Preferred are human patients, dogs, cats and horses. Human patients are female or male person, having an asymptomatic (occult) heart failure, e.g. asymptomatic DCM. As a rule such persons are children, young adults, adults or elderly people with an age of between 6 and 80, preferably between 30 and 65 years.

The term "asymptomatic heart disease" as used hereinabove and hereinbelow relates to any contractile disorder or disease of the heart without clinical symptoms of heart failure.In particular, it relates to heart failure of ISACHC - Stages - Class I. More particular, it relates to a DCM of ISACHC - Stages - Class I.

The term "reduction of the heart size" as used hereinabove and hereinbelow relates to a reduction of the size of the heart of the patient, which is diagnosed using the echocardiography, and having an asymptomatic (occult) heart failure, e.g. asymptomatic DCM.

The term "prolongation until onset of clinical symptoms" as used hereinabove and hereinbelow relates to the time from diagnosing the changes of the heart until the beginning of clinical symptoms of heart failure. In particular, it relates to the prolongation from heart failure of ISACHC Class I to Class II and further to Class III. It more particular relates to the prolongation from heart failure of DCM of ISACHC Class I to Class II and further to Class III.

The term "effective amount" as used herein means an amount sufficient to achieve a prolongation of the time until onet of clinical symptoms and reduction of the heart size when said PDE III inhibitor or Ca²⁺-sensitizing agent is administered in a single dosage form.

According to a further embodiment of the resent invention, the PDE III inhibitor and/or Ca²⁺-sensitizing agent is administered in combination with a second active therapeutic agent. Such a second active therapeutic agent is preferably selected from the group consisting of calcium channel blockers, ACE inhibitors, diuretics, platelet inhibitors, beta blockers and angiotensin II antagonists, aldosterone antagonists, digitalis glycosides, antiarrhythmic agents or diuretics in particular
▪ wherein the calcium channel blocker inhibitor is selected from the group consisting of diltiazem, verapamil and felodipine or a pharmaceutically acceptable derivative thereof; and/or
▪ wherein the ACE inhibitor is selected from the group consisting of omapatrilat, MDL100240, alacepril, benazepril, captopril, cilazapril, delapril, enalapril, enalaprilat, fosinopril, fosinoprilat, imidapril, lisinopril, perindopril, quinapril, ramipril, ramiprilat, saralasin acetate, temocapril, trandolapril, trandolaprilat, ceranapril, moexipril, quinaprilat and spirapril or a pharmaceutically acceptable derivative thereof; and/or
▪ wherein the beta blocker is selected from the group consisting of bisoprolol, carvediol, metoprolol, propranolol and timolol or a pharmaceutically acceptable derivative thereof, and/or
▪ wherein the angiotensin II antagonist is selected from the group consisting of saralasin acetate, candesartan, cilexetil, valsartan, candesartan, losartan potassium, eprosartan, irbesartan, tasosartan, pomisartan and telmisartan or a pharmaceutically acceptable derivative thereof and/or
▪ wherein the aldosterone antagonist is selected from the group consisting of spironolactone, eplerenone, canrenone, potassium canrenone or a pharmaceutically acceptable derivative thereof., and/or
▪ wherein the antiarrhythmic agents are selected from the group consisting of amiodarone, betrylium, disopyramide, dofetilide, flecainide, ibutilide, mexiletine, tocainide, procainamide, propafenone, quinidine, sotalol or a pharmaceutically acceptable derivative thereof., and/or
▪ wherein the diuretic is selected from the group consisting of furosemide, torasemide, bumetanide, etacrynic acid, azosemide, muzolimine, piretanide, tripamide, bendroflumethazide, chlorothiazide, hydrochlorothiazide, hydroflumethiazide, methychlothiazide, polythiazide, trichlormethiazide, chlorthialidone, indapamide, metolazone, quinethazone, etozolin, triamteren, amiloride, or a pharmaceutically acceptable derivative thereof., and/or
▪ wherein the digitalis glycosides is selected from the group consisting of digoxin, digitoxin, g-strophantin, ß-methyldigoxin, ß-acetyldigoxin or a pharmaceutically acceptable derivative thereof.

Most preferably, the PDE III inhibitor or Ca²⁺-sensitizing agent, preferably pimobendan or levosimendan, even more preferred pimobendan is administered together with one or more medicaments selected from the group consisting of one or more ACE-inhibitors, one or more diuretics and one or more digitalis glycosides.

The compounds of this invention can be administered in such oral dosage forms as tablets, capsules (each of which includes sustained release or timed release formulations), pills, powders, granules, elixirs, tinctures, suspensions, syrups, and emulsions. They may also be administered in intravenous (bolus or infusion), intraperitoneal, subcutaneous, or intramuscular form, all using dosage forms well known to those of ordinary skill in the pharmaceutical arts. They can be administered alone, but generally will be administered with a pharmaceutical carrier selected on the basis of the chosen route of administration and standard pharmaceutical practice.

The dosage regimen for the compounds of the present invention will, of course, vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. A physician or veterinarian can determine and prescribe the effective amount of the drug required to prevent, counter, or arrest the progress of the disorder.

By way of general guidance, the daily oral dosage of each active ingredient, preferably of pimobendan or levosimendan, when used for the indicated effects, will range between about 10µg/kg to10 mg/kg, preferably from 0.05 mg/kg to 5 mg/kg, in particular from 0.1 mg/kg to 2 mg/kg. Most preferably from about 0.1 mg/kg to 1.5 mg/kg of pimobendan are administered per day.

The PDE III inhibitors and/or Ca²⁺-sensitizing agents may be administered in a single daily dose, or the total daily dosage may be administered in divided doses of two, three, or four times daily.

The PDE III inhibitors and/or Ca²⁺-sensitizing agents can be administered in intranasal form via topical use of suitable intranasal vehicles, or via transdermale routes, using transdermale skin patches. When administered in the form of a transdermale delivery system, the dosage administration will, of course, be continuous rather than intermittent throughout the dosage regimen.

The PDE III inhibitors and/or Ca²⁺-sensitizing agents are typically administered in admixture with suitable pharmaceutical diluents, excipients, or carriers (collectively referred to herein as pharmaceutical carriers) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with an oral, non-toxic, pharmaceutically acceptable, inert carrier such as lactose, starch, sucrose, glucose, methyl cellulose, magnesium stearate, dicalcium phosphate, calcium sulfate, mannitol, sorbitol and the like; for oral administration in liquid form, the oral drug components can be combined with any oral, non-toxic, pharmaceutically acceptable inert carrier such as ethanol, glycerol, water, and the like. Moreover, when desired or necessary, suitable binders, lubricants, disintegrating agents, and colouring agents can also be incorporated into the mixture. Suitable binders include starch, gelatine, natural sugars such as glucose or beta-lactose, corn sweeteners, natural and synthetic gums such as acacia, tragacanth, or sodium alginate, carboxymethylcellulose, polyethylene glycol, waxes, and the like. Lubricants used in these dosage forms include sodium oleate, sodium stearate, magnesium stearate, sodium benzoate, sodium acetate, sodium chloride, and the like. Disintegrators include, without limitation, starch, methyl cellulose, agar, bentonite, xanthan gum, and the like.

The PDE III inhibitors and/or Ca²⁺-sensitizing agents can also be administered in the form of liposome delivery systems, such as small unilamellar vesicles, large unilamellar vesicles, and multilamellar vesicles. Liposomes can be formed from a variety of phospholipids, such as cholesterol, stearylamine, or phosphatidylcholines.

The PDE III inhibitors and/or Ca²⁺-sensitizing agents may also be coupled with soluble polymers as targetable drug carriers. Such polymers can include polyvinylpyrrolidone, pyran copolymer, polyhydroxypropylmethacrylamide-phenol, polyhydroxyethylaspart-amidephenol, or polyethyleneoxidepolylysine substituted with palmitoyl residues.

Furthermore, the PDE III inhibitors and/or Ca²⁺-sensitizing agents may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacylates, and cross linked or amphipathic block copolymers of hydrogels.

Dosage forms (pharmaceutical compositions) suitable for administration may contain from about 1 milligram to about 100 milligrams of active ingredient per dosage unit.

In these pharmaceutical compositions the active ingredient will ordinarily be present in an amount of about 0.5-95% by weight based on the total weight of the composition.

Gelatine capsules may contain the active ingredient and powdered carriers, such as lactose, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain colouring and flavouring to increase patient acceptance.

In general, water, suitable oil, saline, aqueous dextrose (glucose), and related sugar solutions and glycols such as propylene glycol or polyethylene glycols are suitable carriers for parenteral solutions. Solutions for parenteral administration preferably contain a water soluble salt of the active ingredient, suitable stabilizing agents, and if necessary, buffer substances. Antioxidizing agents such as sodium bisulfite, sodium sulfite, or ascorbic acid, either alone or combined, are suitable stabilizing agents. Also used are citric acid and its salts and sodium EDTA. In addition, parenteral solutions can contain preservatives, such as benzalkonium chloride, methyl-or propyl-paraben, and chlorobutanol.

Suitable pharmaceutical carriers are described in Reminqton's Pharmaceutical Sciences, Mack Publishing Company, a standard reference text in this field.

Where two or more of the foregoing second therapeutic agents are administered with the PDE III inhibitor and/or Ca²⁺-sensitizing agent, generally the amount of each component in a typical daily dosage and typical dosage form may be reduced relative to the usual dosage of the agent when administered alone, in view of the additive or synergistic effect of the therapeutic agents when administered in combination.

Particularly when provided as a single dosage unit, the potential exists for a chemical interaction between the combined active ingredients. For this reason, when the compound of formula 1 and a second therapeutic agent are combined in a single dosage unit they are formulated such that although the active ingredients are combined in a single dosage unit, the physical contact between the active ingredients is minimized (that is, reduced). For example, one active ingredient may be enteric coated. By enteric coating one of the active ingredients, it is possible not only to minimize the contact between the combined active ingredients, but also, it is possible to control the release of one of these components in the gastrointestinal tract such that one of these components is not released in the stomach but rather is released in the intestines. One of the active ingredients may also be coated with a material which effects a sustained-release throughout the gastrointestinal tract and also serves to minimize physical contact between the combined active ingredients.

Furthermore, the sustained-released component can be additionally enteric coated such that the release of this component occurs only in the intestine. Still another approach would involve the formulation of a combination product in which the one component is coated with a sustained and/or enteric release polymer, and the other component is also coated with a polymer such as a low viscosity grade of hydroxypropyl methylcellulose (HPMC) or other appropriate materials as known in the art, in order to further separate the active components. The polymer coating serves to form an additional barrier to interaction with the other component.

Procedures by way of example for preparing the compositions according to the invention will be described in more detail hereinafter. The Examples which follow serve solely as a detailed illustration without restricting the subject matter of the invention. The study was done in symptomatic dogs.

### EXAMPLES

The study which supports the invention is done with pimobendan (Vetmedin®)in dogs suffering of occult dilated cardiomyopathy.

Inclusion criteria were dogs which are diagnosed to have a congestive heart failure of class I according to the ISACHC - score due to dilated cardiomyopathy (DCM). Dogs having a having an asymptomatic (occult) heart failure, e.g. asymptomatic DCM must have reduced contractility of the heart or other signs of reduced cardiac efficacy such as volume overload or ventricle dilatation as confirmed by echocardiography. The dogs stay under mono-therapy until signs of congestive heart failure are developing, deteriorate to heart insufficiency score (ISACHC) class II or III a or b. After confirmation of the congestion by X-ray - radiography the dogs receive furosemid treatment or if the clinical or echocardiografic results makes the addition of another heart failure treatment necessary (primary endpoint). The dogs will be investigated at day 0 prior to first treatment and then approximately 3 month post initiation of the therapy. In order to get long time survival data the investigators' makes then control visits will be repeated every 3 - 6 months. An Echo is performed prior to initiation of therapy (Day 0) and at the following visits to support the clinical diagnosis and to evaluate the effect of the drugs on the contractility of the heart. The echocardiography is performed according to the cardiologic case report form for Irish Wolfhounds The primary parameter investigated will be the heart insufficiency score (ISACHC). Other parameters will be exercise tolerance, findings of the respiratory and circulatory system as well as echocardiography data.

### Results:

After 4 years the interim analysis showed that significant less dogs treated with pimobendan went in the state of clinical symptoms (Class II)

A Left Ventricular Reverse Remodeling (LVRR, = Normalising of the size and function of the left ventricle) has been shown in dogs treated with Pimobendan (ca 80%).

Since other studies in dogs with asymptomatic heart failure with other drugs could not show a prolongation until onset of clinical symptoms these results were unexpected and thus inventive. Furthermore no other study has been shown in dogs with asymptomatic heart disease a reverse remodeling in heart failure and thus the results were unexpected and thus inventive.

### REFERENCES

Calvert, C.A., Chapman, W.C., and Toal, R.C. (1982) Congestive cardiomyopathy in Doberman Pinscher dogs. Journal of the American Veterinary Medical Association 181: 598-602.
Calvert, C.A., Pickus, C.W., Jacobs, G.J., and Brown, J. (1997) Signalment, survival, and prognostic factors in Doberman Pinschers with end-stage cardiomyopathy. Journal of Veterinary Internal Medicine 11: 323-326.
Cohn J N, et al. (2000) Cardiac Remodeling - Concepts and Clinical implications:
A Consensus Paper From an International Forum on Cardiac Remodeling J. of the American College of Cardiology, Vol. 35, No.3, 569-582
Cowley, A.J. and Skene, A.M. (1994) Treatment of severe heart failure: quantity or quality of life? A trial of enoximone. British Heart Journal 72: 226-230.
Ettinger, S.J., Benitz, A.M., Ericsson, G.F., Cifelli, S., Jernigan, A.D., Longhofer, SL, Trimboli, W., and Hanson, P.D. (1998) Effects of enalapril maleate on survival of dogs with naturally acquired heart failure. The Long-Term Investigation of Veterinary Enalapril (LIVE) Study Group. Journal of the American Veterinary Medical Association 213: 1573-1577.
Katz, S.D., Kubo, S.H., Jessup, M., Brozena, S., Troha, J.M., Wahl, J., Cohn, J.N., Sonnenblick, E.H., and LeJemtel, T.H. (1992) A multicenter, randomized, double-blind, placebo-controlled trial of pimobendan, a new cardiotonic and vasodilator agent, in patients with severe congestive heart failure. American Heart Journal 123: 95-103.
Kubo, S.H., Gollub, S., Bourge, R., Rahko, P., Cobb, F., Jessup, M., Brozena, S., Brodsky, M., Kirlin, P., and Shanes, J. (1992) Beneficial effects of pimobendan on exercise tolerance and quality of life in patients with heart failure. Results of a multicenter trial. The Pimobendan Multicenter Research Group. Circulation 85: 942-949.
Monnet, E., Orton, E.C., Salman, M., and Boon, J. (1995) Idiopathic dilated cardiomyopathy in dogs: survival and prognostic indicators. Journal of Veterinary Internal Medicine 9: 12-17.
Kvart C, Haggstrom J, Pedersen HD et al.,(2002) Efficacy of enalapril for prevention of congestive heart failure in dogs with myxomatous valve disease and asymptomatic mitral regurgitation. J Vet Intern Med. 2002 Jan-Feb;16(1):80-8.
Packer, M., Carver, J.R., Rodeheffer, R.J., et al, and for the PROMISE Study Research Group (1991) Effect of oral milrinone on mortality in severe chronic heart failure. New England Journal of Medicine 325: 1468-1475.

The following clauses are also comprised by the underlying invention:
1. The use of a phosphodiesterase type III (PDE III) inhibitor or a Ca²⁺-sensitizing agent or a pharmaceutically acceptable derivative thereof for the preparation of a medication for the reduction of the heart size of a patient having an asymptomatic (occult) heart failure or for the preparation of a medication for the prolongation of the time until onset of clinical symptoms in patients having an asymptomatic (occult) heart failure.
2. The use according to clause 1, wherein the asymptomatic (occult) heart failure is asymptomatic (occult) DCM.
3. The use according to clause 1 and 2, wherein the PDE III inhibitor exhibits additionally calcium sensitising effects.
4. The use according to clause 1 - 3, wherein the PDE III inhibitor or Ca²⁺-sensitizing agent is selected from the group consisting of pimobendan, milrinone, levosimendan, amrinone, enoximone, and piroximone or a pharmaceutically acceptable derivative thereof.
5. The use according to any one of the preceding clauses, wherein the PDE III inhibitor or Ca²⁺-sensitizing agent is utilized in oral or parenteral form.
6. The use according to any one of the preceding clauses, wherein the PDE III inhibitor or Ca²⁺-sensitizing agent is administered in a daily dose from 10µg/kg to10 mg/kg.
7. The use according to any one of the preceding clauses, wherein said patient is a mammal selected from the group consisting of primates including humans, dogs, cats and horses.
8. A method of reduction of the heart size in a patient suffering from heart failure, which method comprises administering to said patient an effective amount of an PDE III inhibitor or Ca²⁺-sensitizing agent or a pharmaceutically acceptable derivative thereof.
9. An article of manufacture comprising packaging material contained within which is a composition effective to reduce the heart size of a patient having an asymptomatic (occult) heart failure and the packaging material comprises a label which indicates that the composition can be used to reduce the heart size of a patient having an asymptomatic (occult) heart failure or to prolongate the time until onset of clinical symptoms in patients having an asymptomatic (occult) heart failure, wherein said composition comprises at least one phosphodiesterase type III (PDE III) inhibitor or Ca²⁺-sensitizing agent or a pharmaceutically acceptable derivative thereof.

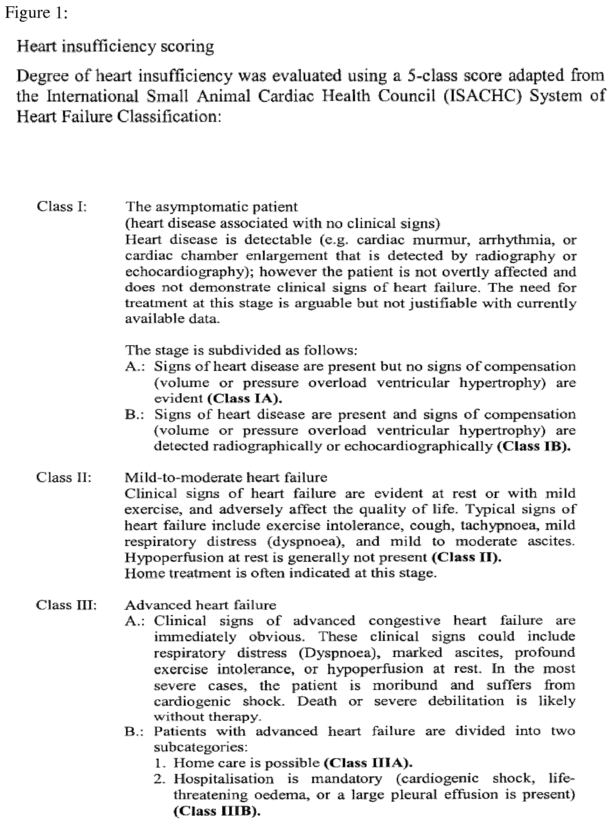

## Claims

1. The use of a phosphodiesterase type III (PDE III) inhibitor and/or a Ca²⁺-sensitizing agent or a pharmaceutically acceptable derivative thereof for the preparation of a medication for the reduction of the heart size of a patient having an asymptomatic (occult) heart failure (ISACHC Class I) and/or for the preparation of a medication for the prolongation of the time until onset of clinical symptoms in patients having an asymptomatic (occult) heart failure (ISACHC Class I).

2. The use of a phosphodiesterase type III (PDE III) inhibitor and/or a Ca²⁺-sensitizing agent or a pharmaceutically acceptable derivative thereof for the preparation of a medication for the reverse remodelling of the heart of a patient with asymptomatic (occult) heart failure (ISACHC Class I) and/or for the preparation of a medication for the delay of onset of clinical symptoms in patients having an asymptomatic (occult) heart failure (ISACHC Class I).

3. The use according to any one of claims 1 to 2, wherein the asymptomatic (occult) heart failure is asymptomatic (occult) DCM.

4. The use according to any one of claims 1 to 3, wherein the PDE III inhibitor exhibits additionally calcium sensitising effects.

5. The use according to any one of claims 1 to 4, wherein the PDE III inhibitor and/or Ca²⁺-sensitizing agent is selected from the group consisting of pimobendan, milrinone, levosimendan, amrinone, enoximone, and piroximone or a pharmaceutically acceptable derivative thereof.

6. The use according to any one of claims 1 to 5, wherein the PDE III inhibitor and/or Ca²⁺-sensitizing agent is utilized in oral or parenteral form.

7. The use according to any one of claims 1 to 6, wherein the PDE III inhibitor and/or Ca²⁺-sensitizing agent is administered in a total daily dose from 10 µg/kg to10 mg/kg, preferably from 0.05 mg/kg to 5 mg/kg, in particular from 0.1 mg/kg to 2 mg/kg.

8. The use according to claim 7, wherein the total daily dose is administered as a single daily dose or as divided doses of two, three, or four times daily.

9. The use according to any one claims 1 to 8, wherein said patient is a mammal or is selected from the group consisting of primates including humans, preferably humans, dogs, cats and horses.

10. The use according to any one claims 1 to 9, wherein the PDE III inhibitor and/or Ca²⁺-sensitizing agent is administered in combination with a second active therapeutic agent.

11. The use according to claim 10, wherein the second active therapeutic agent is selected from the group consisting of calcium channel blockers, ACE inhibitors, diuretics, platelet inhibitors, beta blockers, angiotensin II antagonists, aldosterone antagonists, digitalis glycosides or antiarrhythmic agents.

12. The use according to any one of claims 10 to 11, wherein the PDE III inhibitor and/or Ca²⁺-sensitizing agent is administered together with one or more medicaments selected from the group consisting of one or more ACE inhibitors, one or more diuretics and one or more digitalis glycosides.

13. An article of manufacture comprising packaging material contained within which is a composition effective to reduce the heart size of a patient having an asymptomatic (occult) heart failure and the packaging material comprises a label which indicates that the composition can be used to reduce the heart size of a patient having an asymptomatic (occult) heart failure or to prolongate the time until onset of clinical symptoms in patients having an asymptomatic (occult) heart failure, wherein said composition comprises at least one phosphodiesterase type III (PDE III) inhibitor and/or Ca²⁺-sensitizing agent or a pharmaceutically acceptable derivative thereof.
